# EUROPEAN PATENT APPLICATION

(11) **EP 3 038 023 A1**
(43) Date of publication of application: **29.06.2016**
(21) Application number: 14382562.8
(22) Date of filing: 23.12.2014
(51) Int. Cl.: G06Q 10/04

(54) **A method, a system and computer program products for assessing the behavioral performance of a user**

(71) Applicant: Telefonica Digital España, S.L.U., 28013 Madrid (ES)
(72) Inventor: Matic, Aleksandar, 28013 Madrid (ES); Oliver, Nuria, 28013 Madrid (ES)
(74) Representative: Carlos Hernando, Borja

(57) **Abstract**

The method comprising: a) receiving data (100) regarding behavioral information of a user and relating to a first or a second period of time; b) obtaining a user typical behavioral model (103a) and a user temporary behavioral model (203a) and c) comparing them providing a user behavior deviation; d) selecting a group of individuals for a comparison with the user; e) performing for the individuals steps a) to c) in the same first and second periods of time, and processing the data of the individuals providing a group typical behavioral model (RGR1), a group temporary behavioral model (RGT1) and a group behavioral deviation; f) performing a comparison between the user deviation and the user temporary model (203a) with the group deviation an the group temporary model (RGT1), and using the result to assess the behavioral performance of the user.

## Description

### Field of the invention

The present invention generally relates to methods and systems for behavior assessment of users. In particular, the invention relates to a computer implemented method, a system, and computer program products for assessing the behavioral performance of a user, through the analysis of behavioral parameters of the user and a comparison of the latter with behavioral performance of sets of other users.

### Background of the invention

In order to assess one's behavior (e.g. in the context of mental or physical wellbeing), the standard practice relies on periodical self-reports that suffer from several shortcomings, including memory dependence, recall bias, subjectivity and influence of the current mood of an individual. In case of mental conditions, patients typically visit doctors when the crisis has already happened thus reporting limited information about precursors and making it impossible to eventually prevent the crisis onset. In this regard, the ability to automatically monitor behavior (relying on wearable devices, mobile phone sensing and similar) has shown the potential to overcome the limitations of self-reporting methods that have been used for years in health related sciences as well as in clinical practice.

However, providing solely behavioral parameters extracted from sensor readings without a broader context can provide limited information for assessing individuals' behavior or understanding significant behavioral changes, mainly for two reasons, 1) the reasons for changes in one's behavior are often complex to understand, particularly to distinguish whether the changes happened due to the personal reasons or due to external contextual factors (such as a weather change, a specific event, etc.), 2) the difficulty in establishing a reference point for the behavior considered to be "normal" or "baseline" for a monitored patient.

The data collected through mobile phones has been leveraged to recognize mood [1] and stress [2], to understand determinants of mood changes [3], and to help manage stress, anxiety and mood disturbances [4]. A few studies have investigated the feasibility of providing a smartphone-based platform to continuously acquire behavioral data of bipolar disorder patients to detect significant changes in their behavior related to maniac and depressive episodes. In this line, there are also a few very recent off-the-shelf mobile applications (e.g. PRIORI [6]) that analyze voice and/or behavioral parameters to detect the phases in bipolar disorder. Yet, the majority of mobile phone applications that are designed to track behavior in the context of mental health still rely on self-reports, whereas a minority of mHealth apps that take the advantage of automatic monitoring of behavior, such as Ginger.io or Mobilyze, provide a rather simplistic behavioral assessment by signaling changes in one's behavior (e.g. staying at home for several days) or presenting solely inferred behavioral parameters to the specialists. At a more generic level, the increasingly popular wearable fitness trackers (such as Fitbit, Jawbone and many similar devices) assess one's physical activity behavior by comparing it to his/her own base-line routine or general guidelines.

Several patents have been proposed to automatically assess behavior and detect significant behavioral changes. For instance, patent application US-A1-2014/213938 discloses systems and methods to determine changes in the individual patterns by analyzing and parsing information related to human lifestyle. The input related to human lifestyle considers one or more body sensors.

Patent application US-A1-2011/184250 provides early warning method and system for chronic disease management, placing the focus on respiratory diseases such as asthma. The method comprises a) receiving information related to expect future patient's activity, b) predicting expected local ambient conditions in patient's surrounding, c) predicting health outcomes, d) intervening by sending a message to the patient of a member of his/her care network.

Patent application US-A1-2011/118555 discloses a system and methods for screening, treating and monitoring psychological conditions in a remote manner. Patients are in constant communication with one or more healthcare staff that controls the competition of health programs. The health professionals review and analyze the collected data for making assessments of the patient's mental health status.

Patent application EP-A2-2660745 discloses mental health digital behavior monitoring system and method that track the use of mobile phones, tablets and/or similar devices, detect irregularities in patients' behavioral patterns and provides a deterioration prediction. The behavior is analysed with respect to the base line behavior of a monitored individual without regards of other individuals' behavior.

However, the current solutions for automatic behavioral monitoring and analysis either report behavioral performance regardless of its assessment or in some cases they assess one's behavior and wellbeing status by comparing it to his/her typical behavior or general guidelines (i.e. what is a considered to be a "normal" behavior, such as a number of steps, calories burnt, mobility patterns, etc.). Performing behavioral assessment in such an isolated manner, and with little regard to the wider context, gives limited information about one's behavior or in certain cases it can be even misleading.

### References:

[1] R. Likamwa, Y. Lu, N. Lane, L. Zhong, "MoodScope: building a mood sensor from smartphone usage patterns", In Proc. of MobiSys, pp 389-402, 2013.
[2] A. Bogomolov, B. Lepri, M. Ferron, F. Pianesi, A.S. Pentland, "Pervasive Stress Recognition for Sustainable Living", IEEE Pervasive and Communications Workshops, 2014.
[3] A. Matic, V. Osmani, A. Popleteev, O. Mayora, "Smartphone Sensing to Examine Effects of Social Interactions and Non-Sendentary Work Style on Mood Changes", In Proc. of CONTEXT, 2011.
[4] V. Harrison, J. Proudfoot, P. P. Wee, G. Parker, D. Pavlovic, V. Manicavasagar, "Mobile Mental Health: Review of the emerging field and proof of concept study", Journal of Mental Health, Vol.20, No.6, pp 509-524, 2011.
[5] http://www.gizmag.com/priori-bipolar-disorder-voice-app/32023/ , [accessed November 2014]

### Description of the Invention

Embodiments of the present invention provide a computer-implemented method for assessing the behavioral performance of a user. Specifically, the assessment is based on the analysis of the user's behavior, his/her base-line behavior and the behavioral trends of a set of other individuals/users, or reference groups, within the same time frame.

To that end, the method, using one or more processors of a computing system, first, a) receives or acquires data regarding behavioral information of a user, said data relating to a first or a second period of time, and b) obtains a user typical ('base-line') behavioral model by processing extracted features characterizing a repetitive conduct of the user from the received data related to first period of time, and a user temporary behavioral model by processing extracted features characterizing a temporary conduct of the user from the received data related to a second period of time. Once the two behavioral models of the user are obtained, the method c) compares them to provide a user behavior deviation.

After that, d) a group of individuals whose behaviors are to be used as reference points for a comparison with the user are selected and divided into at least one group category. Then, the method e) performs for the individuals included in said at least one group category the previous described steps a) to c) in the same first and second periods of time, and processes the data of the individuals included in said at least one group providing a group typical behavioral model, a group temporary behavioral model and a group behavioral deviation established through a comparison between them.

Finally, f) a comparison between the user behavioral deviation and the user temporary behavior model with the group behavioral deviation and the group temporary behavior model is performed. The result of said comparison is used to assess the behavioral performance of the user.

According to the invention, the first period of time is of a longer duration than the second period of time. For instance, the first period of time may comprise duration of several months or several years and the second period of time may comprise, for example, duration of one day, one or more weeks or one month.

According to an embodiment related to mental health monitoring, an alarm to a healthcare staff or to a care network of the user is sent if a significant behavioral irregularity relevant for the mental condition of the user is detected in said assessment. For example, the alarm may comprise an audible and/or a visual sound. Besides, the alarm may also be outputted in a mobile computing device or an online platform.

At least one group category can be selected according to: a contact relationship in a communication network, the same or similar geographical location, the same or similar demographics, the same or similar daily activity patterns, the same or similar mobility patterns, or the same or similar disease, among others, of the individuals with the user.

According to an embodiment, the received data in step a) is pre-processed by means of applying at least one of a noise reduction technique, an anonymization technique, a data cleaning technique, or a resampling technique, and aligned in time. Additionally, the pre-processed and aligned data may be stored in a database.

The data may be automatically captured by a data acquisition device that may comprise either a sensor device including at least one of a wearable sensor device, a mobile computing device or an ambient sensor device, or alternatively, a processor acquiring digital footprints generated by the user through the usage of electronic devices, said footprints including data captured from an Internet provider network or a mobile operator network and consisting of communication dynamics, Internet browsing activities and/or mobility patterns.

Other embodiments of the invention that are disclosed herein include a system configured to implement one or more aspects of the disclosed methods, as well as software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

The present invention improves behavioral monitoring and assessment in comparison to the state of the art systems that do not consider a wider context around the monitored user and relevant reference behaviors. Moreover, the present invention allows to evaluate behavior with respect to the user's environment (cultural, geographical, demographical, etc.) in a specific period of time (e.g. behavior in summer vs. behavior in winter) and a better understanding of the causes of behavioral changes i.e. whether the causes are related to personal factors (e.g. due to the mental conditions) or to external context (thus being reflected also in the behavior of other people in the same time period). In addition, the present invention allows reducing the number of false alarms and provides the foundation for building persuasive applications - comparing oneself to others can stimulate positive behavioral changes.

### Brief Description of the Drawings

The previous and other advantages and features will be more fully understood from the following detailed description of embodiments, with reference to the attached drawings, which must be considered in an illustrative and non-limiting manner, in which:
Fig. 1 illustrates some examples of sources of data that can be used as a proxy to the user's behavior.
Fig. 2 illustrates the different steps performed by present invention for building temporal and typical behavioral models, according to different embodiments.
Fig. 3 illustrates how the information is extracted for the behavioral assessment.
Fig.4 illustrates an example of an automatic comparative and contextual behavioral assessment.

### Detailed Description of the Invention

The present invention allows for an automatic monitoring and assessment of users' behavior and detection of significant behavioral changes. The automatically collected data is processed and utilized, by one or more processor of a computing system (not illustrated), to build behavioral models of monitored users as well as of sets of other individuals (referred to as reference groups - RGs). The behavioral assessment is based on the analysis of the users (or patients) temporary behavior, his/her base-line behavior (or typical behavior) and the behavioral trends of his/her RGs within the same time frame.

The reference group - RG of individuals whose behavior is aggregated and used for a relative comparison to a monitored user's behavior can include individuals that: are in the network of close contacts, from the same/similar geographical area (district, city, country, etc.), from the same/similar demographics, have the same/similar daily activities (e.g. same company), have the same/similar mobility patterns (e.g. similar commuting routes), share the same symptoms/disease, among others.

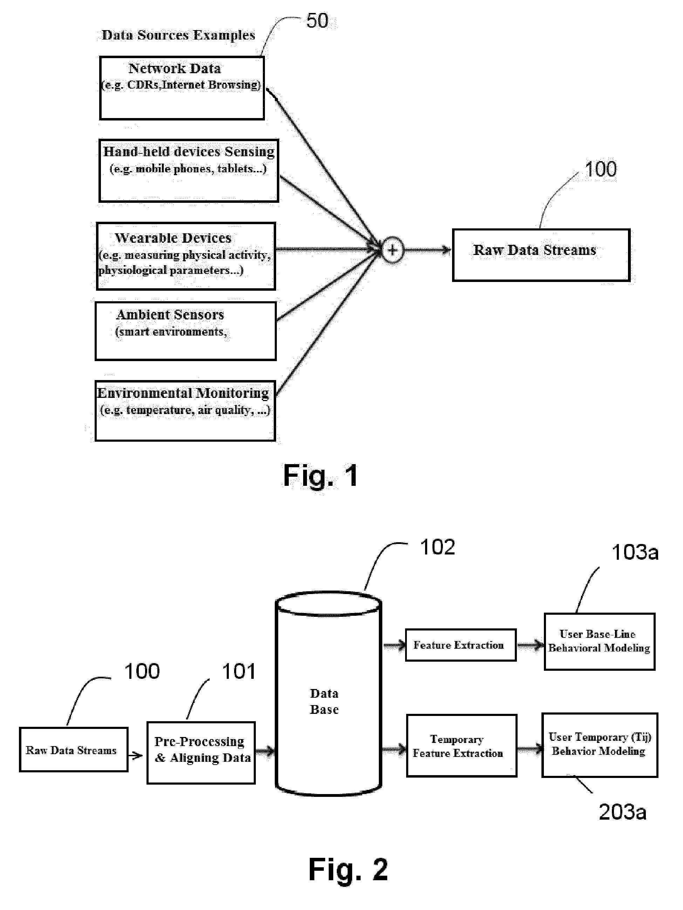

The data that is used as a proxy to the user's behavioral patterns can be captured from sensors (including wearable sensors, mobile phones, ambient sensors, etc.), or from a network (the digital footprints generated through the usage of electronic devices, such as the data captured from Internet provider network or mobile operator network and consisted of communication dynamics, Internet browsing activities and mobility patterns). Fig. 1 illustrates some examples of data sources that can be used as a proxy to the user's behavior.

With reference to Fig. 2, are illustrated the different steps that can be performed by the proposed method for building temporal 203a and base-line behavioral 103a models of a user. As the data 100 from one or multiple sources 50 arrives, preferably (although it is not always necessary) it is being pre-processed 101. Depending on the kind of data different pre-processing may be required such as noise reduction, anonymization, data cleaning, resampling, etc.). Then, the pre-processed data is aligned in time and can be stored in a database 102, or in a temporary buffer.

The behavior of a monitored user can be modeled for at least two, first and second, different periods of time. In principle, the invention compares two behavioral models - one that is related to a user's base-line behavior 103a (preferably computed over a longer period of time, or first period of time, such as several months, several years or a time period defined by a specific embodiment - e.g. behavior during summer), and the other one that is related to a user's temporary behavior 203a (preferably computed over a shorter time period, or second period of time, such as one day, a weekend, one or more weeks or even a variable period defined by a specific moment - *Tij* from the moment *i* until the moment *j*).

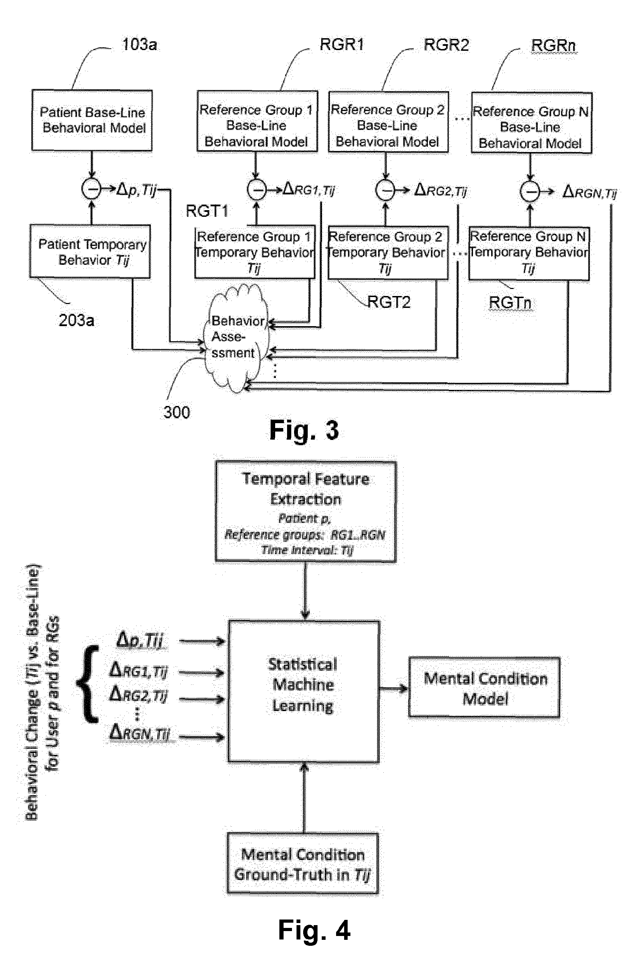

Fig. 3 illustrates the extracting information steps used for the behavioral assessment 300. For a user, the temporary behavior 203a in a specific time frame *Tij* is compared to his/her established base-line behavior 103a for determining significant changes. In comparison to the prior-art, present invention involves the comparison of the current behavior 203a and its deviation from the base-line of a monitored subject (Δ*_{p,Tij}*) for the specific period of time *Tij* with the current behavior RGTx, x = 1, 2, ..., n, and its deviation of one or more reference groups (Δ*_{RG,Tij}*) in the same time interval.

The user's behavior and behavioral changes can be characterized with one variable or a set of variables i.e. features extracted from the collected data using the state of the art methods. Quantifying the behavioral change of the reference groups (Δ*_{RC}*, *_{Tij}*) can include different methods and it depends on the types of the considered variables. For instance, one possibility may be by considering the distribution of a certain variable across individuals that belong to one reference group and comparing it to the value of the same variable related to the user's temporary behavior.

The behavioral assessment 300 of a user during a certain time period *Tij* is based on the analysis of his/her current behavior 203a, deviation of the behavior in *Tij* with respect to the user's typical (base-line) behavior, as well as on the current behavior of one or more reference groups, RGTx, x = 1, 2, ..., n, in the same time period Tij and the relative behavioral change of one or more reference groups with respect to the base-line behavior of the individuals in these groups. This information can be used to provide an automatic behavioral assessment and/or mental condition inference and/or physical condition inference. In addition, said information may be delivered to a user or to a human supervisor (such as healthcare staff) for better understanding of the behavior of a monitored user.

### Human supervised behavioral assessment

Using the extracted information of Fig. 3, the behavioral assessment 300 in a certain short time period *Tij* can be provided by a human such as a personal coach, therapist, a doctor, or even by a user him/herself. In addition to a temporary behavior assessment, the inferred base-line behavior of the related reference groups can provide better understanding of the typical behavior in the user's environment (cultural, geographical, demographical, a group of similar patients, etc.) as opposed to or complementary to the general guidelines related to the recommended behavior.

### Automatic behavioral assessment

By using temporary features related to the behavior of a user and the reference group in a time frame *Tij,* the deviation of behavior within *Tij* with respect to the base-line behavior (for both a user and the reference groups) combined with the ground-truth information about a user's mental condition within *Tij,* the proposed method assesses the user's behavior and learns a mental condition model that can be used to automatically infer mental health status using the extracted information (Fig. 4).

In case of detected irregularities in the user's temporary behavior and the patterns that correspond to the precursors to the crisis or the crisis onset (specifically in case of mental health conditions), the present invention provides a signal that can trigger an alarm to formal or informal healthcare network around the user or to the user him/herself.

### Additional Applications

Different representations of the inferred behavior of a user and the comparison to the reference groups can be used to provide persuasive applications for stimulating positive behavioral changes.

The behavior of the reference groups within the time frame Tij provides a proxy of the contextual information around a user that can affect his/her behavior without a need to know the exact reason of a specific behavior. For instance, if a user decreased a number of outgoing calls/SMS but the same trend was perceived among his colleagues the reason can lie in the context at the company such as a close deadline; if a user decreased his/her mobility but the same patterns was inferred for an increased number of individuals in the same city, the reason could lie in the weather or a season change; on the other hand, if no important behavioral changes are detected in the reference groups but only in the user, there is a higher probability that the reason lies at the user's individual level (e.g. related to his/her mental state).

While the foregoing is directed to embodiments of the present invention, other and further embodiments of the invention may be devised without departing from the basic scope thereof. For example, other aspects may be implemented in hardware or software or in a combination of hardware and software.

Additionally, the software programs included as part of the invention may be embodied in a computer program product that includes a computer useable medium. For example, such a computer usable medium can include a readable memory device, such as a hard drive device, a flash memory device, a CD-ROM, a DVD/ROM, or a computer diskette, having computer readable program code segments stored thereon. The computer readable medium can also include a communications link, either optical, wired, or wireless, having program code segments carried thereon as digital or analog signals.

The scope of the present invention is determined by the claims that follow.

## Claims

1. A computer implemented method for assessing the behavioral performance of a user, the method comprising using one or more processors of a computing system performing following steps:
a) receiving data (100) regarding behavioral information of a user, said data (100) relating to a first and/or a second period of time;
b) obtaining:
b1) a user typical behavioral model (103a) by processing extracted features characterizing a repetitive conduct of the user from the received data related to first period of time, and
b2) a user temporary behavioral model (203a) by processing extracted features characterizing a temporary conduct of the user from the received data related to a second period of time;
c) comparing said obtained user typical behavior model (103a) with said user temporary behavior model (203a) providing a user behavior deviation;
d) selecting a group of individuals whose behaviors are to be used as reference points for a comparison with the user and dividing the selected group of individuals into at least one group category;
e) performing for the individuals included in said at least one group category steps a) to c) in the same first and second periods of time, and processing the data of the individuals included in said at least one group providing a group typical behavioral model (RGR1), a group temporary behavioral model (RGT1) and a group behavioral deviation established through a comparison between them;
f) performing a comparison between the user behavioral deviation and the user temporary behavior model (203a) with the group behavioral deviation an the group temporary behavior model (RGT1), and using the result of said comparison to assess the behavioral performance of the user.

2. The method of claim 1, wherein said first period of time is of a long duration of said second period of time, the first period of time comprising a duration of at least one to several months or several years and the second period of time comprising a duration from one day to a month.

3. The method of claim 1, further comprising sending an alarm to a healthcare staff or to a care network of the user if a significant behavioral irregularity of the user relevant for a mental condition of the user is detected in said assessment.

4. The method of claim 3, wherein said alarm comprises an audible and/or a visual sound.

5. The method of claims 3 or 4, wherein the alarm is outputted in a mobile computing device.

6. The method of claim 1, wherein the at least one group category being selected at least according to:
- a contact relationship in a communication network;
- a same or similar geographical location;
- a same or similar demographics;
- a same or similar daily activity;
- same or similar mobility patterns; or
- a same disease,
of the individuals with the user.

7. The method of claim 1, wherein said received data (100) in step a) is pre-processed (101) by means of applying at least one of a noise reduction technique, an anonymization technique, a data cleaning technique, or a resampling technique, and aligned in time.

8. The method of claim 7, further comprising storing in a database (102) the pre-processed and aligned data.

9. The method of claim 1 or 8, wherein said data (100) being automatically captured by a data acquisition device comprising a sensor device including at least one of a wearable sensor device, a mobile computing device or an ambient sensor device.

10. The method of claim 1 or 8, wherein said data (100) being automatically captured by a data acquisition device comprising a processor acquiring digital footprints generated by the user through the usage of electronic devices, said footprints including data captured from an Internet provider network or a mobile operator network and consisting of communication dynamics, Internet browsing activities and/or mobility patterns.

11. A system for assessing behavioral performance of a user, the system comprising:
- at least one processor; and
- a memory including instructions that, when executed by the at least one processor cause the processor to implement a method according to any of claims 1 to 10.

12. The system of claim 11, wherein a data acquisition device, comprising a sensor device including at least one of a wearable sensor device, a mobile computing device or an ambient sensor device, is configured for capturing said data (100).

13. The system of claim 11, wherein a data acquisition device, comprising a processor acquiring digital footprints generated by the user through the usage of electronic devices, said footprints including data captured from an Internet provider network or a mobile operator network and consisting of communication dynamics, Internet browsing activities and/or mobility patterns, is configured for capturing said data (100).

14. A computer program product comprising software program code instructions which when loaded into a computer system controls the computer system to perform each of the methods according to any one of claims 1 to 10.
